# EUROPEAN PATENT SPECIFICATION

(11) **EP 2 377 483 B1**
(45) Date of publication and mention of the grant of the patent: **21.08.2013**
(21) Application number: 08878837.7
(22) Date of filing: 17.12.2008
(51) Int. Cl.: A61B 5/01, A61B 19/02, A61B 19/08, B65D 5/72, B65D 83/00, B65D 85/62, G01J 5/02

(54) **DISPENSING CONTAINER BOX FOR EAR THERMOMETER SHEATH**
SPENDEBEHÄLTERSCHACHTEL FÜR OHRTHERMOMETERSCHUTZKAPPEN
BOITE D'EMBALLAGE POUR LA DISTRIBUTION D'EMBOUTS DE PROTECTION POUR THERMOMETRE AURICULAIRE

(43) Date of publication of application: 19.10.2011
(73) Proprietor: Actherm Inc., Hsinchu 30078 (TW)
(72) Inventor: LI, Liangyi, Jhubei City Hsinchu County 302 Taiwan (CN)
(74) Representative: Lang, Christian
(86) International application number: PCT/CN2008/002024
(87) International publication number: WO 2010/069093

(56) References cited:
- CN-Y- 2 433 223
- JP-A- 2000 041 955
- JP-A- 2005 288 190
- TW-U- M 333 172
- TW-U- M 333 560
- US-A1- 2004 016 766
- US-A1- 2007 160 412

## Description

### BACKGROUND OF THE INVENTION

### 1. Technical Field

The present invention relates to a dispensing container for probe covers of ear thermometers and a manufacturing method of such dispensing container. More particularly, the present invention relates to a dispensing container that facilitates dispensing and assembly of probe covers to a measuring probe of an ear thermometer and a manufacturing method of such dispensing container.

### 2. Description of Related Art

The conventional dispensing container for storing and providing probe covers of ear thermometers is not convenient in use because its users, e.g., medical workers, have to take out the probe covers from the dispensing container and then assemble the probe covers to the measuring probes of ear thermometers manually. Particularly, the infrared ear thermometers need to work with probe covers to prevent infection and maintain cleanness of the measuring probes. Although some prior art devices, such as those disclosed in US Patent No. 4,993,424, US Patent No. 5,100,018, and US Patent No. 6,840,402, enable automatic feed for assembling probe covers to ear thermometers and have been used in medical facilities, their mechanical complexity and bulkiness nevertheless inconvenience users in both installing the probe covers into the feeding mechanisms and dispatching the probe covers from the feeding mechanisms. Besides, there is a commercially available dispensing container for probe covers that has a lateral side to be torn open so as to form an opening through which an ear thermometer gets access to the probe covers stored in the dispensing container. However, this dispensing container is disadvantaged by its complicated operation and loss of package integrity once the opening is formed, which exposes the probe covers to the air and, consequently, to contamination.

The prior art is also disclosed in JP 2005288190 A, JP 2000 041955 A, TW 333 172 U, TW 333 560 U and CN 2433223 Y.

### SUMMARY OF THE INVENTION

In an attempt to remedy the shortcomings of the prior art devices, the present invention provides a dispensing container for probe covers of ear thermometers and a manufacturing method of such dispensing container. The dispensing container comprises a hollow main body and a bottom portion. The hollow main body has a first opening at its one end, and the probe covers are placed in the dispensing container in such a way that each probe cover has its open end facing the first opening. The hollow main body further has a second opening at another end opposite to the end having the first opening. At least a pair of first abutting portions are provided inside the hollow main body adjacent to the second opening. The bottom portion has one end inside the hollow main body and an opposite end jutting out of the second opening of the hollow main body. The bottom portion has a third opening facing the first opening for receiving hollow bodies of the probe covers. The bottom portion is provided exteriorly with at least a pair of second abutting portions adjacent to the third opening for abutting on the first abutting portions so as to prevent the bottom portion from coming off through the second opening.

Hence, a primary objective of the present invention is to provide a dispensing container for probe covers of ear thermometers and a manufacturing method of such dispensing container, wherein the dispensing container has a hollow main body formed with first abutting portions for abutting on second abutting portions formed on a bottom portion of the dispensing container so that the bottom portion is retained to avoid coming off from the hollow main body.

A secondary objective of the present invention is to provide a dispensing container for probe covers of ear thermometers and a manufacturing method of such dispensing container, wherein the dispensing container has a bottom portion formed with third abutting portions for abutting on a flange of the nearest probe cover so as to retain the stacked probe covers in the dispensing container.

Another objective of the present invention is to provide a dispensing container for probe covers of ear thermometers and a manufacturing method of such dispending container, wherein the dispensing container has a hollow main body and a bottom portion both made of a flexible material so that an interference formed between an exterior surface of the bottom portion and an interior surface of the hollow main body, thereby enabling the dispensing container to be shrinkable in size.

Yet another objective of the present invention is to provide a dispensing container for probe covers of ear thermometers and a manufacturing method of such dispensing container, wherein friction between an exterior surface of a bottom portion and an interior surface of a hollow main body is greater than friction between the interior surface of the hollow main body and flanges of the probe covers. Therefore, when a user directly inserts an ear thermometer into the dispensing container, a measuring probe of the ear thermometer presses the hollow main body downward and makes the probe covers move relatively upward so that the ear thermometer is assembled with the nearest probe cover smoothly.

Still another objective of the present invention is to provide a dispensing container for probe covers of ear thermometers and a manufacturing method of such dispensing container, wherein the dispensing container has a bottom portion formed with a transversely extended shoulder portion that abuts on a periphery of a central opening formed at a bottom of a hollow main body of the dispensing container so that the bottom portion is retained to avoid coming off the hollow main body.

A further objective of the present invention is to provide a dispensing container for probe covers of ear thermometers and a manufacturing method of such dispensing container, wherein the dispensing container has a bottom portion formed with a shoulder portion that abuts on a flange of the nearest probe cover so that the stacked probe covers are stably held in the dispensing container.

Another objective of the present invention is to provide a dispensing container for probe covers of ear thermometers and a manufacturing method of such dispensing container, wherein an interference is formed between a shoulder portion formed at a bottom portion of the dispensing container and an interior surface of a hollow main body of the dispensing container so that the dispensing container is shrinkable in size.

Still another objective of the present invention is to provide a dispensing container for probe covers of ear thermometers and a manufacturing method of such dispensing container, wherein friction between a shoulder portion at a bottom portion of the dispensing container and an interior surface of a hollow main body of the dispensing container is greater than friction between the interior surface of the hollow main body and flanges of the probe covers. Therefore, when a user directly inserts an ear thermometer into the dispensing container, a measuring probe of the ear thermometer presses the hollow main body downward and makes the probe covers move relative upward so that the ear thermometer is assembled with the nearest probe cover smoothly.

The present invention further provides a dispensing container for probe covers of ear thermometers and a manufacturing method of such dispensing container. The dispensing container is configured to receive a plurality of probe covers and comprises a hollow main body and a bottom portion, wherein the hollow main body enables the dispensing container to receive the plurality of probe covers stacked together. The hollow main body has a first opening at its one end, and the probe covers are placed in the dispensing container in such a way that each probe cover has its open end facing the first opening. The hollow main body further has a bottom at another end opposite to the end having the first opening. A pair of open portions are formed at two lateral sides of the hollow main body, respectively. The bottom portion, which is received in the hollow main body, is smaller than or equal to the hollow main body in length and movable inside the hollow main body. A pair of handle portions are provided at two lateral sides of the bottom portion, respectively, so that when the bottom portion is received in the hollow main body, the handle portions jut out of the open portions of the hollow main body, respectively. The bottom portion has a third opening facing the first opening for receiving hollow bodies of the probe covers.

Hence, another objective of the present invention is to provide a dispensing container for probe covers of ear thermometers and a manufacturing method of such dispensing container, wherein the dispensing container has a bottom portion received in a hollow main body of the dispensing container and smaller than or equal to the hollow main body in length, thereby allowing more probe covers to be stored in the dispensing container. Meanwhile, a user can directly insert an ear thermometer into the dispensing container to fetch a probe cover and assemble the probe cover to the ear thermometer.

Yet another objective of the present invention is to provide a dispensing container for probe covers of ear thermometers and a manufacturing method of such dispensing container, wherein friction between an exterior surface of a bottom portion of the dispensing container and an interior surface of a hollow main body of the dispensing container is greater than friction between the interior surface of the hollow main body and flanges of the probe covers. Therefore, a user can directly insert an ear thermometer into the dispensing container to fetch a probe cover and assemble the probe cover to the ear thermometer regardless of the size of a measuring probe of the ear thermometer.

Still another objective of the present invention is to provide a dispensing container for probe covers of ear thermometers and a manufacturing method of such dispensing container, wherein the dispensing container has handle portions each having an ear formed as a bent element to facilitate holding and force exertion by a user.

A further objective of the present invention is to provide a dispensing container for probe covers of ear thermometers and a manufacturing method of such dispensing container, wherein the dispensing container has a skirt portion formed by handle portions and peripherally encircling a hollow main body of the dispensing container to facilitate holding and force exertion by a user.

Another objective of the present invention is to provide a dispensing container for probe covers of ear thermometers and a manufacturing method of such dispensing container, wherein the dispensing container has a skirt portion whose lower edge is coupled with a bracket so that a user can operate the dispensing container without holding the same in hand.

### BRIEF DESCRIPTION OF THE DRAWINGS

The invention as well as a preferred mode of use, further objectives, and advantages thereof will be best understood by reference to the following detailed description of illustrative embodiments when read in conjunction with the accompanying drawings, wherein:
Fig. 1A is a perspective view of a dispensing container according to a first preferred embodiment of the present invention, wherein a hollow main body and a bottom portion of the dispensing container are depicted;
Fig. 1B is a top perspective view of a hollow main body according to another aspect of the first preferred embodiment of the present invention;
Fig. 1C is a perspective view of the dispensing container according to the first preferred embodiment of the present invention, showing how the hollow main body and the bottom portion are assembled;
Fig. 1D is a bottom perspective view of the bottom portion according to the first preferred embodiment of the present invention;
Fig. 2 is a perspective view of the dispensing container according to the first preferred embodiment of the present invention, showing how the hollow main body, the bottom portion, and a cover case are assembled;
Fig. 3 is a perspective view of the dispensing container according to the first preferred embodiment of the present invention, wherein the hollow main body, the bottom portion, and the cover case are assembled;
Fig. 4 is a perspective view of a dispensing container according to a third preferred embodiment of the present invention, wherein a hollow main body and a bottom portion are depicted in an assembled state;
Fig. 5A is a perspective view of the hollow main body according to the third preferred embodiment of the present invention;
Fig. 5B is a perspective view of the bottom portion according to the third preferred embodiment of the present invention;
Fig. 6 is a perspective view of the dispensing container according to the third preferred embodiment of the present invention, wherein the bottom portion is received in the hollow main body;
Fig. 7A is a perspective view of a dispensing container according to a fifth preferred embodiment of the present invention;
Fig. 7B is a perspective view of a bottom portion of the dispensing container according to the fifth preferred embodiment of the present invention;
Fig. 7C is a side perspective view of the bottom portion of the dispensing container according to the fifth preferred embodiment of the present invention;
Fig. 7D is a perspective view of a dispensing container according to another aspect of the fifth preferred embodiment of the present invention, wherein the dispensing container comprises a bottom portion formed with ears;
Fig. 7E is a perspective view of a bottom portion of a dispensing container according to yet another aspect of the fifth preferred embodiment, wherein the bottom portion is provided with bent elements;
Fig. 7F is a perspective view of a dispensing container according to still another aspect of the fifth preferred embodiment of the present invention, wherein the dispensing container comprises a bottom portion formed with a skirt portion; and
Fig. 7G is another perspective view of the dispensing container of Fig. 7F, wherein the dispensing container is settled on a bracket.

### DETAILED DESCRIPTION OF THE PREFERRED EMBODIMENTS

While the present invention proposes a dispensing container for probe covers of ear thermometers and a manufacturing method of such dispensing container, the basic principles of ear thermometers are known to one of ordinary skill in the art and therefore are not be discussed at any length herein. Meanwhile, the accompanying drawings referred to in the following description are not made to scale and need not be so because they are provided only to illustrate structural features of the present invention schematically.

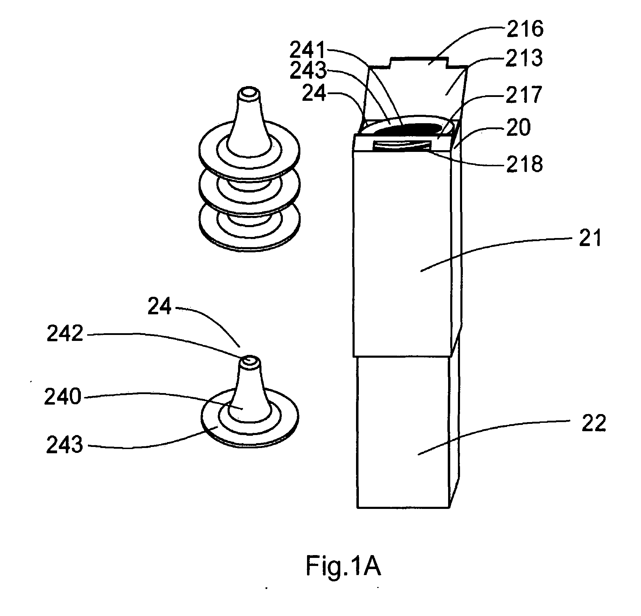

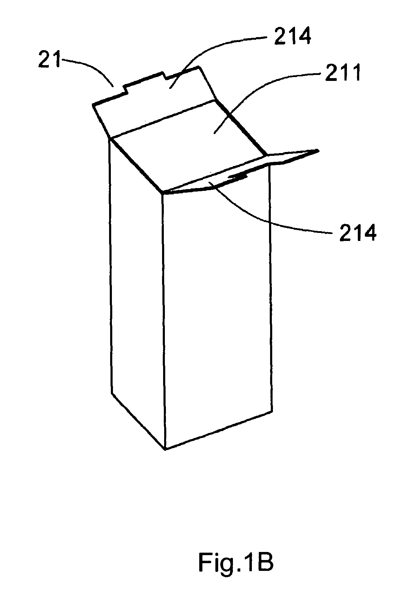

Please refer to Fig. 1A for a dispensing container 20 for probe covers of ear thermometers according to a first preferred embodiment of the present invention. The dispensing container 20 is configured to receive a plurality of probe covers 24 each having a hollow body 240 with one open end 241 and an opposite closed end 242, wherein a flange 243 is extended radially outward from the open end 241. The dispensing container 20 comprises a hollow main body 21 and a bottom portion 22. The hollow main body 21 enables the dispensing container 20 to receive the plural probe covers 24 stacked together. Referring to Fig. 1B, a first opening 211 is formed at one end of the hollow main body 21, and the probe covers 24 are placed in the hollow main body 21 in such a way that the open ends 241 of the probe covers 24 face the first opening 211 of the hollow main body 21.

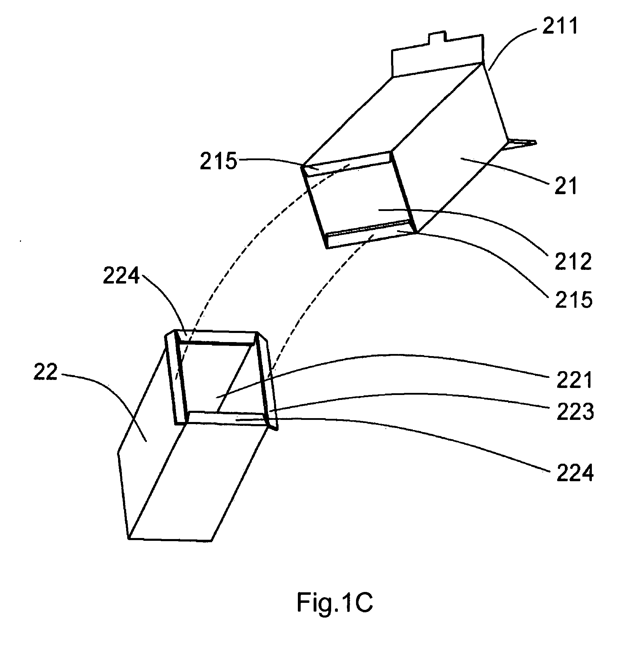

As can be seen in Fig. 1C, a second opening 212 is formed at another end of the hollow main body 21 opposite to the end having the first opening 211. A pair of first abutting portions 215 are provided adjacent to the second opening 212. In addition, one end of the bottom portion 22 that faces the second opening 212 of the hollow main body 21 (as indicated by the broken lines) is formed with a third opening 221. A pair of second abutting portions 223 are provided adjacent to the third opening 221. When the bottom portion 22 is inserted through the second opening 212 of the hollow main body 21 along the direction indicated by the broken lines of Fig. 1C so as to fit in the hollow main body 21, the second abutting portions 223 abut on the first abutting portions 215 to prevent the bottom portion 22 from coming off the hollow main body 21. Furthermore, a pair of third abutting portions 224 are also provided adjacent to the third opening 221 of the bottom portion 22 for abutting on the flange 243 of the nearest probe cover 24 so as to stably hold the stacked probe covers 24 in the bottom portion 22. Each of the first abutting portions 215, the second abutting portions 223, and the third abutting portions 224 are of a strip-like structure.

In the present embodiment, the hollow main body 21 and the bottom portion 22 are both made of a flexible material and have substantially similar sectional shapes. In virtue of frictional interference between an exterior surface of the bottom portion 22 and an interior surface of the hollow main body 21, wherein friction between the exterior surface of the bottom portion 22 and the interior surface of the hollow main body 21 is greater than friction between the interior surface of the hollow main body 21 and the flanges 243 of the probe covers 24, a user can insert an ear thermometer (not shown in the drawings) directly into the dispensing container 20 to make the bottom portion 22 move further into the hollow main body 21 so that one said probe cover 24 is successfully retrieved. Meantime, as the bottom portion 22 moves further into the hollow main body 21, the dispensing container 20 is reduced in size as a whole.

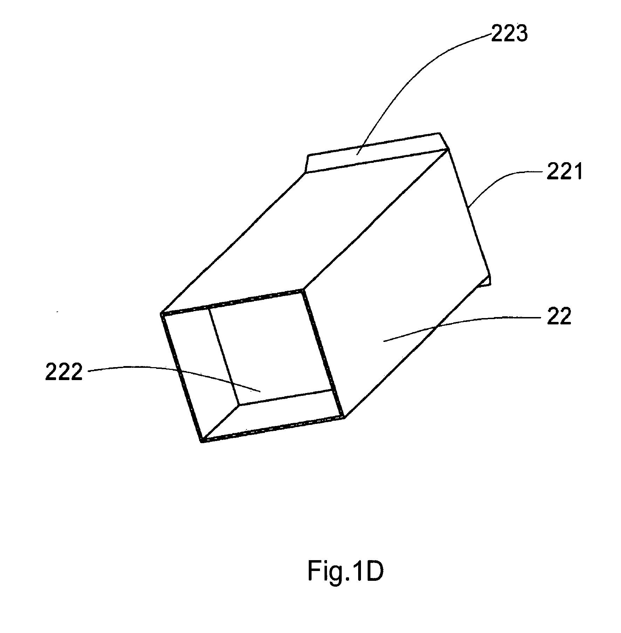

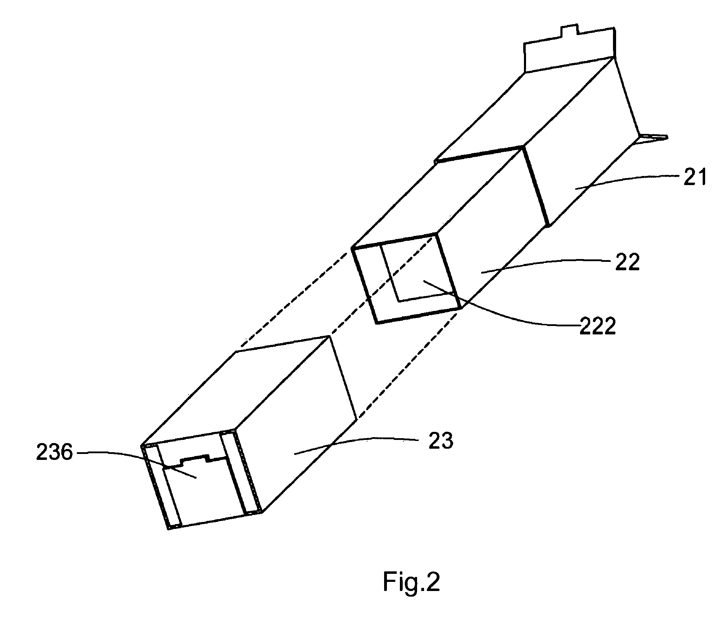

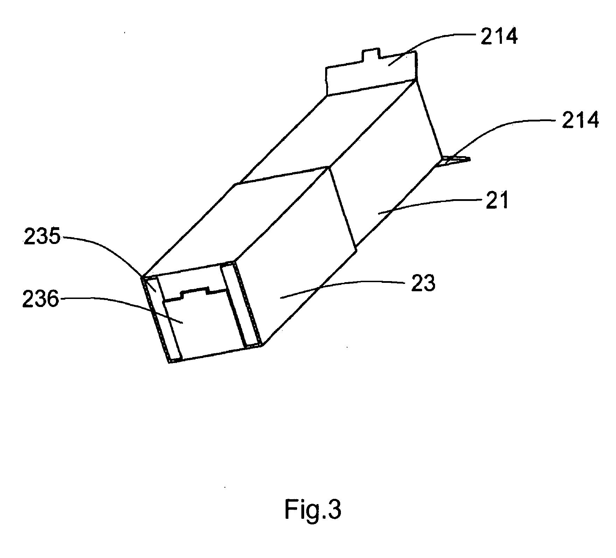

Referring to Figs. 2 and 3, the dispensing container 20 further comprises a cover case 23 enclosing the bottom portion 22 for enhanced convenience in retrieving the probe covers 24 from the dispensing container 20. As the probe covers 24 stored in the dispensing container 20 are taken out successively, the bottom portion 22 is pushed deeper into the hollow main body 21 so that the length of the bottom portion 22 jutting out of the hollow main body 21 reduces accordingly. Now that the bottom portion 22 is enclosed by the cover case 23, even when a user is holding the cover case 23 and trying to retrieve the probe covers 24 from the dispensing container 20, the hollow main body 21 is capable of being pushed downward without having his/her hand being jammed by the hollow main body 21. Thus, the cover case 23 is smoothly fit into the hollow main body 21 by the bottom portion 22. Besides, an accommodating space 222 is formed at a bottom of the bottom portion 22, as shown in Fig. 1D, for partially accommodating a bracket that supports the dispensing container 20. In addition, the cover case 23 has a bottom provided with a lower lid 236 for bearing the bottom portion 22.

Referring to Figs. 2 and 3, in order to assemble the dispensing container 20 to the bracket, the cover case 23 is firstly mounted around the bottom portion 22 so that the bottom of the bottom portion 22 abuts on the lower lid 236 of the cover case 23. Then, the bracket pierces through a pair of lateral holes 235 formed on the cover case 23 so as to reach the accommodating space 222 of the bottom portion 22. Particularly, the cover case 23 is substantially equal to the bottom portion 22 in length while the bottom of the cover case 23 and the hollow main body 21 have substantially similar peripheral shapes. The cover case 23 is made of paper or plastic. Moreover, the sectional shape of the hollow main body 21 may be circular, triangular, trapezoidal, or polygonal, instead of the depicted rectangular shape.

Referring back to Figs. 1A and 1B, the bottom portion 22 and the hollow main body 21 have substantially equal lengths. An upper lid 213 and a flap 217 are provided opposite to each other adjacent to the first opening 211 of the hollow main body 21 for closing the first opening 211 and thus preventing the probe covers 24 from escaping through the first opening 211. Therein, a protruding portion 216 is formed at one side of the upper lid 213 while a retaining opening 218 is formed at a center of the flap 217 so that the upper lid 213 is coupled with the flap 217 by piercing the protruding portion 216 of the upper lid 213 through the retaining opening 218 of the flap 217. The hollow main body 21 and the bottom portion 22 are made of paper or plastic. As shown in Fig. 1B, the upper lid 213 is not limited to a single-piece configuration but formed as a pair of cover plates 214. The two cover plates 214 are respectively provided with a protruding portion and a notch corresponding to the protruding portion so that when the protruding portion and the notch are coupled together, the two cover plates 214 jointly close the first opening 211. Of course, the coupling between the cover plates 214 can be implemented in different manners depending on practical applications.

A second preferred embodiment of the present invention is herein proposed to provide a method for manufacturing the dispensing container as described in the first preferred embodiment. The method comprises the following steps:
(1) providing a flexible hollow main body which has one end formed with a first opening and an opposite end formed with a second opening, wherein at least a pair of first abutting portions are provided inside the hollow main body adjacent to the second opening, and the hollow main body is configured to receive a plurality of stacked probe covers such that open ends of the probe covers face the first opening of the hollow main body, in which the first opening of the hollow main body is formed with an upper lid for closing the first opening, the upper lid being optionally composed of two cover plates for closing the first opening ;
(2) providing a flexible bottom portion which has one end received in the hollow main body and an opposite end jutting out of the second opening of the hollow main body, wherein a third opening is formed at the end of the bottom portion that faces the first opening for receiving hollow bodies of the probe covers, and the bottom portion is exteriorly provided with at least a pair of second abutting portions adjacent to the third opening for abutting on the first abutting portion so as to prevent the bottom portion from coming off through the second opening, the bottom portion being formed with at least a pair of third abutting portions adjacent to the third opening for abutting on a flange of the nearest probe cover, the end of the bottom portion opposite to the third opening being formed as a bottom, the bottom portion and the hollow main body having substantially similar sectional shapes, an interference being formed between an exterior surface of the bottom portion and an interior surface of the hollow main body; and
(3) further providing a cover case, wherein the cover case and the bottom portion have substantially equal lengths, and a bottom of the cover case and the hollow main body have substantially similar peripheral shapes, the bottom of the cover case being provided with a lower lid for bearing the bottom portion.

In the present embodiment, the hollow main body, the bottom portion, the cover case, and other related elements of the dispending container are the same as those of the first preferred embodiment in structure, material, manner of coupling, and operation.

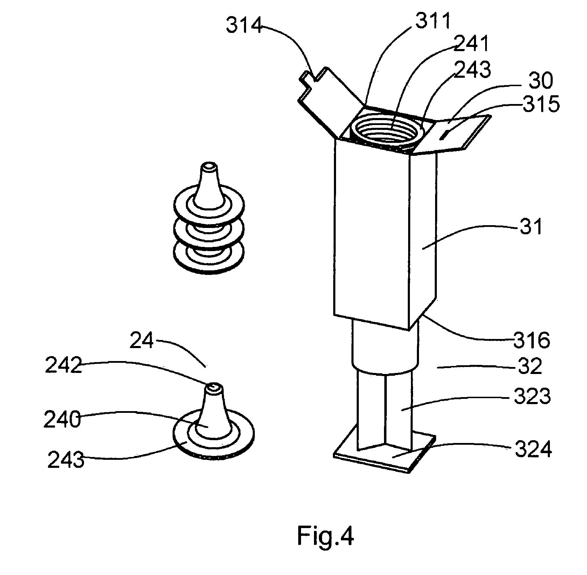

Please refer to Fig. 4 for a dispensing container 30 for probe covers of ear thermometers according to a third preferred embodiment of the present invention. The dispensing container 30 is configured to receive a plurality of probe covers 24 each having a hollow body 240 with one open end 241 and an opposite closed end 242, wherein a flange 243 is extended radially outward from the open end 241. The dispensing container 30 comprises a hollow main body 31 and a bottom portion 32. The hollow main body 31 enables the dispensing container 30 to receive the plural probe covers 24 stacked together. A first opening 311 is formed at one end of the dispensing container 30, and the probe covers 24 are placed in the dispending container 30 in such a way that the open ends 241 of the probe covers 24 face the first opening 311 of the hollow main body 31.

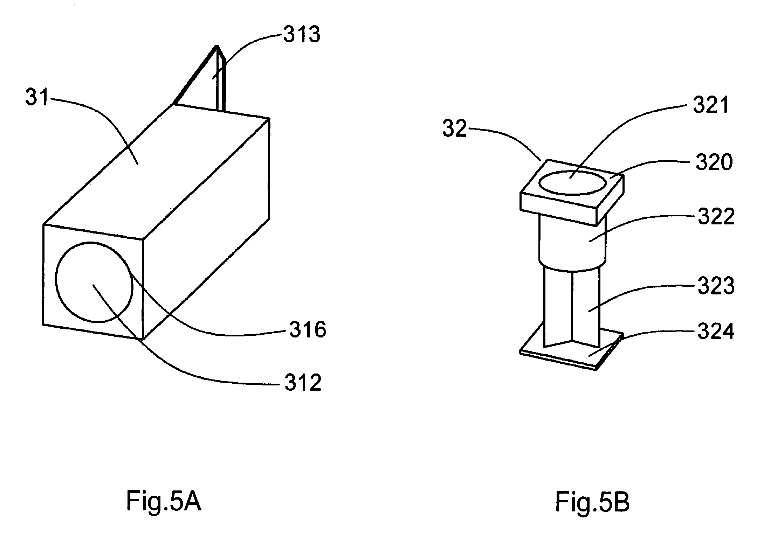

As can be seen in Figs. 5A and 5B, a bottom 316 is formed at another end of the hollow main body 31 opposite to the end having the first opening 311 and is centrally formed with a second opening 312. The bottom portion 32 has one end received in the hollow main body 31 and an opposite end jutting out of the second opening 312 of the hollow main body 31. The bottom portion 32 has a third opening 321 facing the first opening 311 for receiving the hollow bodies 240 of the probe covers 24. A shoulder portion 320 is extended transversely from the third opening 321 of the bottom portion 32 for abutting on the flange 243 of the nearest probe cover 24 so that the stacked probe covers 24 are stably held in the bottom portion 32. Besides, the shoulder portion 320 of the bottom portion 32 also abuts on the bottom 316 of the hollow main body 31 so as to prevent the bottom portion 32 from coming off through the second opening 312 of the hollow main body 31.

According to Fig. 4, the hollow main body 31 and the bottom portion 32 are both made of a flexible material so that the bottom portion 32 is allowed to fit into the hollow main body 31. Meanwhile, an interference is formed between the shoulder portion 320 of the bottom portion 32 and an interior surface of the hollow main body, wherein friction between the shoulder portion 320 of the bottom portion 32 and the interior surface of the hollow main body 31 is greater than friction between the interior surface of the hollow main body 31 and the flanges 243 of the probe covers 24. Thus, a user can insert an ear thermometer (not shown in the drawings) directly into the dispensing container 30 to make the bottom portion 32 move toward the first opening 311 of the hollow main body 31 so that one said probe cover 24 is successfully retrieved. At the same time, as the bottom portion 32 moves further into the hollow main body 31, the dispensing container 30 is reduced in size as a whole.

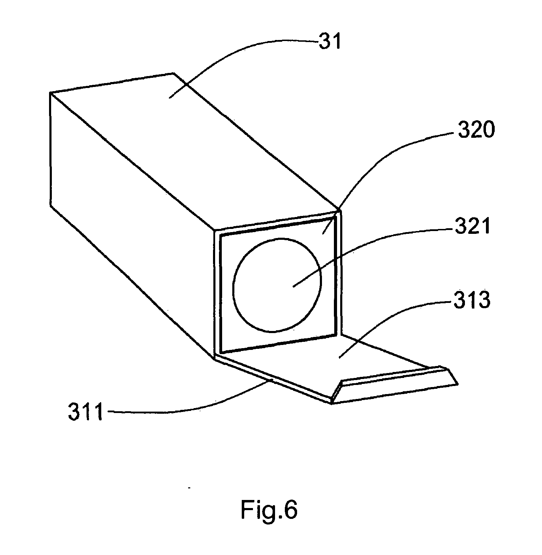

Referring to Fig. 6, the shoulder portion 320 of the bottom portion 32 and the first opening 311 of the hollow main body 31 have substantially similar sectional shapes while the bottom portion 32 and the hollow main body 31 have substantially equal lengths. Although the hollow main body 31 is shown in the drawings as having a rectangular sectional shape, the sectional shape of the hollow main body 31 may also be circular, triangular, trapezoidal, or polygonal. The hollow main body 31 and the bottom portion 32 are made of paper or plastic. Moreover, an upper lid 313 is provided adjacent to the first opening 311 of the hollow main body 31 so as to close the first opening 311 and thus prevent the probe covers 24 from escaping from the dispensing container 30.

Referring back to Fig. 4, the upper lid 313 is not limited to a single-piece configuration but composed of a pair of cover plates 314, 315. The two cover plates 314, 315 are provided respectively with a protruding portion and a slot corresponding to the protruding portion so that when the protruding portion of the cover plate 314 is coupled with the slot of the cover plate 315 the cover plates 314, 315 jointly close the first opening 311. Of course, the coupling between the cover plates 314, 315 can be implemented in different manners depending on practical applications. Referring to Fig. 5B, a tubular portion 322 is extended from the third opening 321 of the bottom portion 32 for receiving the probe covers 24. Furthermore, a detachable pedestal 324 is transversely provided at the end of the bottom portion 32 opposite to the third opening 321. A connecting rod 323 having a sectional shape like a three-pointed star is provided between the tubular portion 322 and the pedestal 324 for evenly distributing the pressure applied to the bottom portion 32 by the dispensing container 30. In addition to the three-pointed star configuration, the connecting rod 323 may alternatively be of a tubular shape that extends all the way to the pedestal 324. Moreover, the pedestal 324 and the hollow main body 31 have substantially similar peripheral shapes.

A fourth preferred embodiment of the present invention is herein proposed to provide a method for manufacturing the dispensing container as described in the third preferred embodiment. The method comprises the following steps:
(1) providing a flexible hollow main body which has one end formed with a first opening and an opposite end formed as a bottom centrally provided with a second opening, the hollow main body being configured to receive a plurality of stacked probe covers such that open ends of the probe covers face the first opening of the hollow main body;
(2) providing a bottom portion which has one end received in the hollow main body and an opposite end jutting out of the second opening of the hollow main body, wherein the bottom portion has a third opening facing the first opening for receiving hollow bodies of the probe covers, and a shoulder portion is extended transversely outward from the third opening for abutting on a flange of the nearest probe cover, the shoulder portion also abutting on the bottom of the hollow main body so as to prevent the bottom portion from coming off through the second opening of the hollow main body, the bottom portion being made of a material more rigid than a material of which the hollow main body is made so that an interference is formed between the shoulder portion of the bottom portion and an interior surface of the hollow main body;
(3) further providing a pedestal transversely at the end of the bottom portion that is opposite to the end having the third opening, wherein the pedestal and the hollow main body have substantially similar sectional shapes; and
(4) further providing at least an upper lid adjacent to the first opening of the hollow main body for closing the first opening, wherein the upper lid may be composed of a pair of cover plates for closing the first opening.

In the present embodiment, the hollow main body, the bottom portion, and other related elements of the dispensing container are the same as those of the third preferred embodiment in structure, material, manner of coupling, and operation.

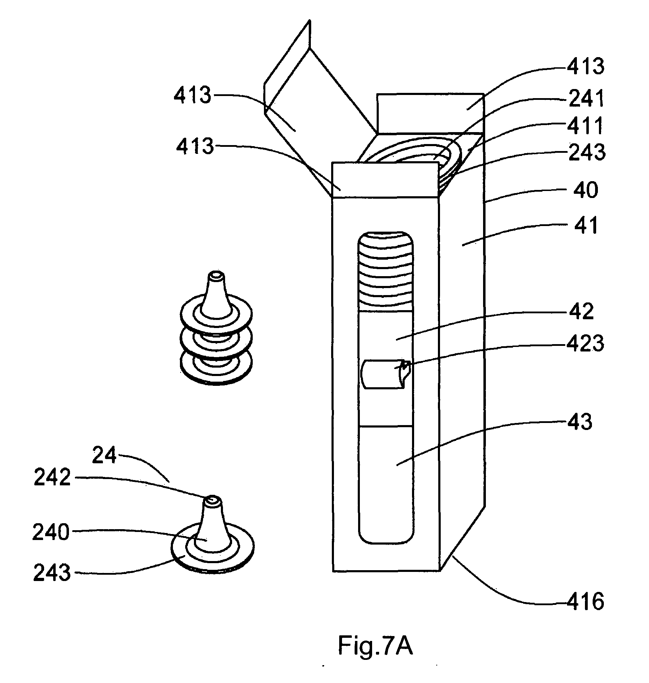

Please refer to Fig. 7A for a dispensing container 40 for probe covers of ear thermometers according to a fifth preferred embodiment of the present invention. The dispensing container 40 is configured to receive a plurality of probe covers 24 each having a hollow body 240 with one open end 241 and an opposite closed end 242, wherein a flange 243 is extended radially outward from the open end 241. The dispensing container 40 comprises a hollow main body 41 and a bottom portion 42. The hollow main body 41 enables the dispensing container 40 to receive the plural probe covers 24 stacked together. A first opening 411 is formed at one end of the hollow main body 41, and the probe covers 24 are placed in the hollow main body 41 in such a way that the open ends 241 of the probe covers 24 face the first opening 411 of the hollow main body 41. Besides, the hollow main body 41 has a bottom 416 at another end opposite to the end having the first opening 411 and a pair of open portions 43 formed at two lateral sides of the hollow main body 41, respectively.

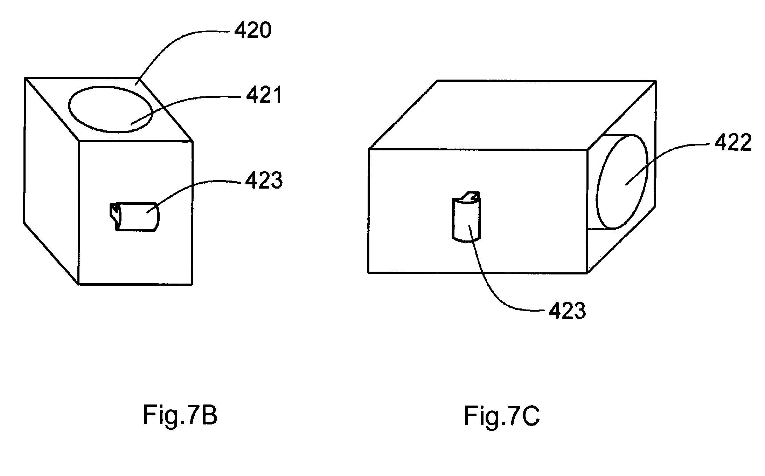

Referring to Figs. 7B and 7C, the bottom portion 42, which is received in the hollow main body 41, is smaller than or equal to the hollow main body 41 in length and is movable inside the hollow main body 41. The bottom portion 42 of the present embodiment has a reduced size so that more probe covers 24 can be stored in the dispensing container 40. The bottom portion 42 has a third opening 421 facing the first opening 411. A tubular portion 422 is extended from the third opening 421 for receiving the hollow bodies of the probe covers 24. A shoulder portion 420 is extended transversely from the third opening 421 for abutting on the flange 243 of the nearest probe cover 24 so that the stacked probe covers 24 are stably held in the bottom portion 42. A pair of handle portions 423 are provided at two lateral sides of the bottom portion 42, respectively, and jutting out of the open portions 43 of the hollow main body 41, respectively, to facilitate holding and force exertion by a user.

In the present invention, the hollow main body 41 and the bottom portion 42 are made of a flexible material, and an interference is formed between the bottom portion 42 and an interior surface of the hollow main body 41. In other words, friction between the bottom portion 42 and the interior surface of the hollow main body 41 is greater than friction between the interior surface of the hollow main body 41 and the flanges 243 of the probe covers 24. Thereby, a user can insert an ear thermometer (not shown in the drawings) directly into the dispensing container 40 to make the bottom portion 42 move further into the hollow main body 41 so that one said probe cover 24 is successfully taken out.

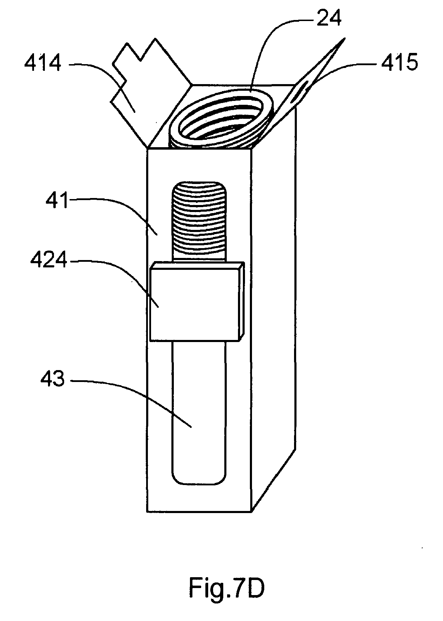

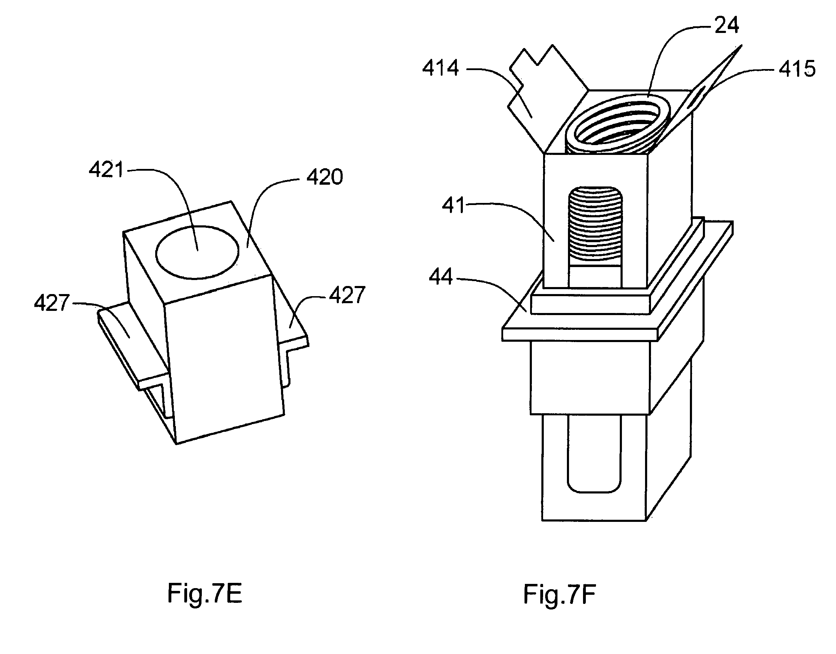

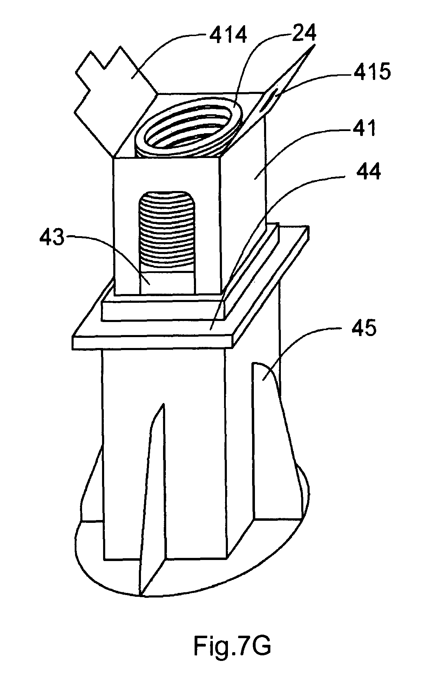

Referring to Fig. 7D, the handle portion 423 of the bottom portion 42 is further extended with an ear 424 which is greater than the open portion 43 of the hollow main body 41 in width so that the user holding the handle portion 423 can easily exert a force to make the bottom portion 42 move smoothly inside the hollow main body 41. Referring to Fig. 7E, the ears 424 are formed as bent elements 427. When a user inserts an ear thermometer (not shown in the drawings) into the dispensing container 40, the bent elements 427 abut on the user's hand and thus facilitate holding and force exertion. Referring to Fig. 7F, the pair of handle portion 423 provided bilaterally on the bottom portion 42 are connected together to form a skirt portion 44 that peripherally encircles the hollow main body 41 to facilitate holding and force exertion by a user. Now seeing Fig. 7G, in order to allow a user to exert a stable force without holding the dispensing container 40 in his/her hand, the skirt portion 44 has its lower edge settled on a bracket 45 so that the dispensing container 40 can be placed stably on a table during use without toppling.

Besides, the bottom portion 42 and the handle portions 423 may be integrally formed as one piece or may be separately made and then assembled, wherein the integrally formed structure is more preferable. The hollow main body 41 or handle portion 423 respectively may have a circular, triangular, trapezoidal, or polygonal sectional shape instead of the depicted rectangular sectional shape. The hollow main body 41, the bottom portion 42, and the handle portions 423 are made of paper or plastic. An upper lid 413 is further provided adjacent to the first opening 411 of the hollow main body 41 for closing the first opening 411 and thus preventing the probe covers 24 from escaping from the dispensing container 40. Alternatively, as can be seen in Fig. 7G, the upper lid 413 is not limited to a one-piece configuration but composed of a pair of cover plates 414, 415. The two cover plates 414, 415 are respectively provided with a protruding portion and a slot corresponding to the protruding portion so that when the protruding portion of the cover plate 414 is coupled with the slot of the cover plate 415, the two cover plates 414, 415 jointly close the first opening 411. Of course, the coupling between the cover plates 414, 415 can be implemented in different manners depending on practical applications.

A sixth preferred embodiment of the present invention is herein proposed to provide a method for manufacturing the dispensing container as described in the fifth preferred embodiment. The method comprises the following steps:
(1) providing a flexible hollow main body which has one end formed with a first opening and an opposite end formed as a bottom, wherein a pair of open portions are formed at two lateral sides of the hollow main body, respectively, the hollow main body being configured to receive a plurality of stacked probe covers such that open ends of the probe covers face the first opening of the hollow main body;
(2) providing a bottom portion that is received in the hollow main body and movable inside the hollow main body, the bottom portion being smaller than or equal to the hollow main body in length, the bottom portion having a third opening facing the first opening for receiving hollow bodies of the probe covers, a shoulder portion being extended transversely from the third opening for abutting on a flange of the nearest probe cover, a pair of handle portions being provided at two lateral sides of the bottom portion, respectively, and jutting out of the open portions of the hollow main body, respectively, wherein an interference is formed between the hollow main body and an interior surface of the bottom portion such that friction between the bottom portion and the interior surface of the hollow main body is greater than friction between the interior surface of the hollow main body and the flanges of the probe covers; and
(3) further providing at least an upper lid adjacent to the first opening of the hollow main body for closing the first opening, wherein the upper lid may be composed of a pair of cover plates for closing the first opening.

In the present embodiment, the hollow main body, the bottom portion, and other related elements of the dispending container are the same as those of the fifth preferred embodiment in structure, material, manner of coupling, and operation.

The present invention has been described by reference to the preferred embodiments and it is understood that the embodiments are not intended to limit the scope of the present invention.

## Claims

1. A dispensing container suitable for probe covers (24) of ear thermometers, each said probe cover (24) having a hollow body (240) with one open end (241) and an opposite closed end (242), wherein a flange (243) is extended radially outward from the open end (241), the dispensing container (20) comprising a hollow main body (21) and a bottom portion (22), the dispensing container (20) being **characterized in**:
the hollow main body (21) being configured to receive a plurality of stacked probe covers (24), a first opening (211) being formed at an end of the hollow main body (21) such that the open ends (241) of the probe covers (24) can face the first opening (211) of the hollow main body, a second opening (212) being formed at another end of the hollow main body (21) opposite to the end having the first opening(211), wherein at least a pair of first abutting portions (215) are provided inside the hollow main body (21) adjacent to the second opening (212); and
the bottom portion (22) having an end received in the hollow main body (21) and an opposite end jutting out of the second opening (212) of the hollow main body (21), the bottom portion (22) having a third opening (221) facing the first opening (211) for receiving the hollow bodies of the probe covers, the bottom portion (22) being provided exteriorly with at least a pair of second abutting portions (223) adjacent to the third opening (221) for abutting on the pair of first abutting portions (215) so as to prevent the bottom portion (22) from coming off through second opening (212), the bottom portion (22) being further provided with at least a pair of third abutting portions (224) adjacent to the third opening(221) for abutting on the flange (243) of the nearest probe cover, the end of the bottom portion (22) opposite to the third opening (221) being formed as a bottom;
wherein the hollow main body (21) and the bottom portion (22) are made of a flexible material and have substantially similar sectional shapes, and an interference is formed between an exterior surface of the bottom portion (22) and an interior surface of the hollow main body (21).

2. The dispensing container of Claim 1, wherein bottom portion (22) and the hollow main body (21) have substantially equal lengths

3. The dispensing container of Claim 1, further comprising a cover case (23), wherein the cover case (23) and the bottom portion (22) have substantially equal lengths while the cover case (23) and the hollow main body (21) have substantiallysimilar peripheral shapes.

4. The dispensing container of Claim 1, wherein the sectional shape of the hollow main body (21) is selected from the group consisting of a rectangular shape, a circular shape, a triangular shape, a trapezoidal shape, and a polygonal shape.

5. A method for making a dispensing container for probe covers of ear thermometers, each said probe cover having a hollow body with an open end and an opposite closed end, wherein a flange is extended radially outward from the open end, and the method comprising steps of:
providing a flexible hollow main body with one end formed with a first opening and an end opposite to the first opening formed with a second opening, at least one pair of first abutting portions being provided inside the hollow main body adjacent to the second opening, and the hollow main body being configured to receive a plurality of stacked probe covers, so that the open ends of the probe covers face the first opening of the hollow main body; and
providing a flexible and soft bottom portion whose one end is received in the hollow main body and an opposite end juts out of the second opening of the hollow main body, the bottom portion having a third opening facing the first opening for receiving the hollow bodies of the probe covers, at least one pair of second abutting portions being provided at an exterior surface of the bottom portion adjacent to the third opening, for abutting against the pair of first abutting portions, so as to prevent the bottom portion from escaping through the second opening, at least one pair of third abutting portions being provided adjacent to the third opening for abutting against the flange of the nearest probe cover, the bottom portion having a bottom at an end opposite to the third opening, wherein the bottom portion and the hollow main body have substantially similar sectional shapes, and an interference being formed between the exterior surface of the bottom portion and an interior surface of the hollow main body.

6. A dispensing containersuitable for probe covers (24) of ear thermometers, each said probe cover having a hollow body (240) with one open end (241) and an opposite closed end (242), wherein a flange (243) is extended radially outward from the open end (241), the dispensing container (30) comprising a hollow main body (31) and a bottom portion (32), the dispensing container (30) being **characterized in**:
the hollow main body (31) being configured to receive the plural stacked probe covers, a first opening (311) being formed at an end of the hollow main body (31) such that the open ends (241) of the probe covers (24) can face the first opening (311) of the hollow main body, a bottom being formed at another end of the hollow main body (31) opposite to the end having the first opening (311), wherein the bottom is centrally formed with a second opening (312); and
the bottom portion (32) having an end received in the hollow main body (31) and an opposite end jutting out of the second opening (312) of the hollow main body (31), the bottom portion (32) having a third opening (321) facing the first opening (311) for receiving the hollow bodies of the probe covers (24), a shoulder portion (320) being transversely extended from the third opening (321) for abutting on the flange (243) of the nearest probe cover (24) and also abutting on the bottom of the hollow main body (31) so as to prevent the bottom portion (32) from coming off through the second opening (312);
wherein the hollow main body (31) and the bottom portion (32) are made of a flexible material, and an interference is formed between the shoulder portion (320) of the bottom portion (32) and an interior surface of the hollow main body (31).

7. The dispensing container of Claim 6, wherein the shoulder portion (320) of the bottom portion (32) and the first opening (311) of the hollow main body (31) have substantially similar shapes.

8. The dispensing container of Claim 6, wherein a pedestal (324) is provided transversely at the end of the bottom portion (32) opposite to the third opening, the pedestal (324) and the hollow main body (31) having substantially similar peripheral shapes, the bottom portion (32) and the hollow main body (31) having substantially equal lengths.

9. The dispensing container of Claim 6, wherein the hollow main body (31) has a sectional shape selected from the group consisting of a rectangular shape, a circular shape, a triangular shape, a trapezoidal shape, and a polygonal shape.

10. A method for making a dispensing container for probe covers of ear thermometers, each said probe cover having a hollow body with an open end and an opposite closed end, wherein a flange is extended radially outward from the open end, and the method comprising steps of:
providing a flexible hollow main body, being configured to receive a plurality of stacked probe covers, a first opening being formed at an end of the hollow main body such that the open ends of the probe covers face the first opening of the hollow main body, a bottom being formed at an end of the hollow main body opposite to the end having the first opening, and a second opening being provided at a center of the bottom; and
providing a bottom portion whose one end is received in the hollow main body and an opposite end juts out of the second opening of the hollow main body, the bottom portion having a third opening facing the first opening for receiving the hollow bodies of the probe covers, a shoulder portion being extended transversely from the third opening for abutting on the flange of the nearest probe cover and the shoulder portion also abutting on the bottom of the hollow main body so as to prevent the bottom portion from escaping through the second opening, and the bottom portion being made of a material more rigid than a material of which the hollow main body is made so that an interference is formed between the bottom portion and an interior surface of the hollow main body.

11. A dispensing container suitable for probe covers (24) of ear thermometers, each said probe cover having a hollow body (240) with an open end (241) and an opposite closed end (242), wherein a flange (243) is extended radially outward from the open end (241), the dispensing container comprising a hollow main body (41) and a bottom portion (42), the dispensing container being **characterized in**:
the hollow main body (41) being configured to receive a plurality of stacked probe covers (24), an opening (411) being formed at an end of the hollow main body (41) such that the open ends (241) of the probe covers (24) can face the opening (411) of the hollow main body (41), a bottom being formed at another end of the hollow main body (41) opposite to the end having the opening (411) of the hollow main body, wherein a pair of open portions (43) are formed at two lateral sides of the hollow main body (41), respectively; and
the bottom portion (42) being received in the hollow main body (41) and being smaller than or equal to the hollow main body (41) in length, wherein the bottom portion (42) is movable inside the hollow main body (41), the bottom portion (42) having an opening (421) facing the opening (411) off the hollow main body for receiving the hollow bodies of the probe covers (24), a shoulder portion (420) being transversely extended from the opening (421) of the bottom portion (42) for abutting on the flange (243) of the nearest probe cover (24), a pair of handle portions (423) being provided at two lateral sides of the bottom portion, respectively, and jutting out of the open portions (43) of the hollow main body (41), respectively; wherein the hollow main body (41) and the bottom portion are made of a flexible material, and an interference is formed between the bottom portion (42) and the interior surface of the hollow main body (41) .

12. The dispensing container of Claim 11, wherein a tubular portion (322) is extended from the third opening (421) of the bottom portion, and an upper lid (413) is provided adjacent to the first opening (411) of the hollow main body (41) for closing the first opening.

13. The dispensing container of Claim 11, wherein each said handle portion (423) of the bottom portion (42) has a shape selected from the group consisting of an arcuate shape, a triangular shape, a rectangular shape, and a polygonal shape.

14. The dispensing container of Claim 11, wherein the hollow main body (41) has a sectional shape selected from the group consisting of a rectangular shape, a circular shape, a triangular shape, a trapezoidal shape, and a polygonal shape.

15. A method for making a dispensing container for probe covers of ear thermometers, each said probe cover having a hollow body with an open end and an opposite closed end, wherein a flange is extended radially outward from the open end, and the method comprising steps of:
providing a flexible hollow main body, being configured to receive a plurality of stacked probe covers, a first opening being formed at an end of the hollow main body such that the open ends of the probe covers face the first opening of the hollow main body, a bottom being formed at an end of the hollow main body opposite to the end having the first opening, and a pair of open portions being formed at two lateral sides of the hollow main body; and
providing a bottom portion that is received in the hollow main body and is smaller than or equal to the hollow main body in length while allowed to move inside the hollow main body, the bottom portion having a third opening facing the first opening for receiving the hollow bodies of the probe covers, a shoulder portion being extended transversely from the third opening for abutting on the flange of the nearest probe cover, a pair of handle portions being provided at two lateral sides of the bottom portion, and the handle portions jutting out of the open portions of the hollow main body, respectively, the bottom portion being made of a material more rigid than a material of which the hollow main body is made so that an interference is formed between the bottom portion and an interior surface of the hollow main body, and a friction between the bottom portion and the interior surface of the hollow main body is greater than a friction between the interior surface of the hollow main body and the flanges of the probe covers.

## Patentansprüche

1. Spenderbehälter, der geeignet ist für Sondenabdeckungen (24) von Ohrther-mometern, wobei jede Sondenabdeckung (24) einen hohlen Körper (240) mit einem offenen Ende (241) und einem gegenüberliegenden geschlossenen Ende (242) aufweist, wobei ein Flansch (243) sich radial von dem offenen Ende (241) nach außen erstreckt, wobei der Spenderbehälter (20) einen hohlen Hauptkörper (21) und einen Bodenbereich (22) umfasst, wobei der Spenderbehälter (20) **dadurch gekennzeichnet ist, dass**
der hohle Hauptkörper (21) darauf ausgelegt ist, eine Vielzahl an gestapelten Sondenabdeckungen (24) aufzunehmen, wobei eine erste Öffnung (211) an einem Ende des hohlen Hauptkörpers (21) ausgebildet ist, so dass die offenen Enden (241) der Sondenabdeckungen (24) auf die erste Öffnung (211) des hohlen Hauptkörpers ausgerichtet sein können, wobei eine zweite Öffnung (212) am anderen Ende des hohlen Hauptkörpers (21) gegenüber dem Ende, das die erste Öffnung (211) aufweist, ausgebildet ist, wobei mindestens ein Paar von ersten Anschlagsbereichen (215) innerhalb des hohlen Hauptkörpers (21) und benachbart zu der zweiten Öffnung (212) bereitgestellt sind; und dass der Bodenbereich (22) ein Ende, das in den hohlen Hauptkörper (21) aufgenommen ist, und ein gegenüberliegendes Ende aufweist, das aus der zweiten Öffnung (212) des hohlen Hauptkörpers (21) hervorsteht, wobei der Bodenbereich (23) eine auf die erste Öffnung (211) ausgerichtete dritte Öffnung (221) zum Aufnehmen der hohlen Körper der Sondenabdeckungen aufweist, wobei der Bodenbereich (22) außen mit mindestens einem Paar von zweiten Anschlagsbereichen (223) benachbart zu der dritten Öffnung (221) zum Anliegen an das Paar von ersten Anschlagsbereichen (215) versehen ist, um den Bodenbereich (22) davon abzuhalten, durch die zweiter Öffnung (212) hindurch zu rutschen, wobei der Bodenbereich (22) ferner mit mindestens einem Paar von dritten Anschlagsbereichen (224) benachbart zu der dritten Öffnung (221) zum Anliegen an dem Flansch (243) der nächstgelegenen Sondenabdeckung versehen ist, wobei das Ende des Bodenbereichs (22) gegenüber der dritten Öffnung (221) als ein Boden ausgebildet ist;
wobei der hohle Hauptkörper (21) und der Bodenbereich (22) aus einem flexiblen Material hergestellt sind und im Wesentlichen ähnliche Schnittformen aufweisen und wobei ein Eingriff zwischen einer äußeren Oberfläche des Bodenbereichs (22) und einer inneren Oberfläche des hohlen Hauptkörpers (21) ausgebildet ist.

2. Spenderbehälter nach Anspruch 1, wobei der Bodenbereich (22) und der hohle Hauptkörper (21) im Wesentlichen gleich große Längen aufweisen.

3. Spenderbehälter nach Anspruch 1, der ferner ein Abdeckungsgehäuse (23) umfasst, wobei das Abdeckungsgehäuse (23) und der Bodenbereich (22) im Wesentlichen gleiche Längen aufweisen, während das Abdeckungsgehäuse (23) und der hohle Hauptkörper (21) im wesentlichen ähnliche Umfangsformen aufweisen.

4. Spenderbehälter nach Anspruch 1, wobei die Schnittform des hohlen Hauptkörpers (21) aus der Gruppe ausgewählt ist, die aus einer Rechteckform, einer Kreisform, einer Dreiecksform, einer Trapezform und einer Polygonalform besteht.

5. Verfahren zur Herstellung eines Spenderbehälters für Sondenabdeckungen von Ohrthermometern, wobei jede Sondenabdeckung einen hohlen Körper mit einem offenen Ende und einem gegenüberliegenden geschlossenen Ende aufweist, wobei ein Flansch sich radial von dem offenen Ende nach außen erstreckt, wobei das Verfahren die Schritte umfasst:
Bereitstellen eines flexiblen hohlen Hauptkörpers mit einem Ende, das mit einer ersten Öffnung ausgebildet ist, und einem Ende gegenüber der ersten Öffnung, das mit einer zweiten Öffnung ausgebildet ist, wobei mindestens ein Paar von ersten Anschlagsbereichen innen in dem hohlen Hauptkörper benachbart zu der zweiten Öffnung bereitgestellt sind und wobei der hohle Hauptkörper darauf ausgelegt ist, eine Vielzahl an gestapelten Sondenabdeckungen aufzunehmen, so dass das offene Ende der Sondenabdeckungen auf die erste Öffnung des hohlen Hauptkörpers ausgerichtet ist; und
Bereitstellen eines flexiblen und weichen Bodenbereichs, dessen eines Ende in dem hohlen Hauptkörper aufgenommen ist und dessen anderes Ende aus der zweiten Öffnung des hohlen Hauptkörpers hervarsteht, wobei der Bodenbereich eine auf die erste Öffnung ausgerichtete dritte Öffnung zum Aufnehmen der hohlen Körper der Sondenabdeckungen aufweist, wobei mindestens ein Paar von zweiten Anschlagsbereichen an einer äußeren Oberfläche des Bodenbereichs benachbart zu der dritten Öffnung zum Anliegen an das Paar von ersten Anschlagsbereichen bereitgestellt ist, um den Bodenbereich davon abzuhalten, durch die zweiter Öffnung hindurch zu rutschen, wobei mindestens ein Paar von dritten Anschlagsbereichen benachbart zu der dritten Öffnung zum Anliegen an dem Flansch der nächstgelegenen Sondenabdeckung bereitgestellt ist, wobei der Bodenbereich einen Boden an einem Ende gegenüber der dritten Öffnung aufweist, wobei der Bodenbereich und der hohle Hauptkörper im Wesentlichen ähnliche Schnittformen aufweisen und wobei ein Eingriff zwischen der äußeren Oberfläche des Bodenbereichs und einer inneren Oberfläche des hohlen Hauptkörpers ausgebildet ist.

6. Spenderbehälter, der geeignet ist für Sondenabdeckungen (24) von Ohrthermometern, wobei jede Sondenabdeckung einen hohlen Körper (240) mit einem offenen Ende (241) und einem gegenüberliegenden geschlossenen Ende (242) aufweist, wobei ein Flansch (243) sich radial von dem offenen Ende (241) nach außen erstreckt, wobei der Spenderbehälter (30) einen hohlen Hauptkörper (31) und einen Bodenbereich (32) umfasst, wobei der Spenderbehälter (30) **dadurch gekennzeichnet ist, dass**
der hohle Hauptkörper (31) darauf ausgelegt ist, die Vielzahl gestapelter Sondenabdeckungen aufzunehmen, wobei eine erste Öffnung (311) an einem Ende des hohlen Hauptkörpers (31) ausgebildet ist, so dass das offene Ende (241) der Sondenabdeckungen (24) auf die erste Öffnung (311) des hohlen Hauptkörpers ausgerichtet sein können, wobei ein Boden gegenüber dem ersten Ende, das die erste Öffnung (311) aufweist, an einem anderen Ende des hohlen Hauptkörpers (31) ausgebildet ist, wobei der Boden zentral mit einer zweiten Öffnung (312) ausgebildet ist; und dass
der Bodenbereich (32) ein Ende, das in dem hohlen Hauptkörper (31) aufgenommen ist, und ein gegenüberliegendes Ende aufweist, das aus der zweiten Öffnung (312) des hohlen Hauptkörpers (31) hervorsteht, wobei der Bodenbereich (32) eine dritte Öffnung (321), die auf die erste Öffnung (311) ausgerichtet ist, zum Aufnehmen der hohlen Körper der Sondenabdeckungen (24), und einen Schulterbereich (320) aufweist, der sich transversal von der dritten Öffnung (321) zum Anliegen an dem Flansch (243) der nächstgelegenen Sondenabdeckung (24) und auch zum Anliegen an dem Boden des hohlen Hauptkörper (31) erstreckt, um so zu verhindern, dass der Bodenbereich (32) durch die zweite Öffnung (312) hindurch fällt,
wobei der hohle Hauptkörper (31) und der Bodenbereich (32) aus einem flexiblen Material hergestellt sind und wobei ein Eingriff zwischen dem Schulterbereich (320) des Bodenbereichs (32) und einer inneren Oberfläche des hohlen Hauptkörpers (31) ausgebildet ist.

7. Spenderbehälter nach Anspruch 6, wobei der Schulterbereich (320) des Bodenbereichs (32) und die erste Öffnung (311) des hohlen Hauptkörpers (31) im Wesentlichen ähnliche Formen aufweisen.

8. Spenderbehälter nach Anspruch 6, wobei ein Sockel (324) transversal an dem Ende des Bodenbereichs (32) und gegenüber der dritten Öffnung bereitgestellt ist, wobei der Sockel (324) und der hohle Hauptkörper (31) im Wesentlichen ähnliche Umfangsformen aufweisen, wobei der Bodenbereich (32) und der hohle Hauptkörper (31) im Wesentlichen gleiche Längen aufweisen.

9. Spenderbehälter nach Anspruch 6, wobei der hohle Hauptkörper (31) eine Schnittform aufweist, die aus der Gruppe ausgewählt ist, die aus einer Rechteckform, einer Kreisform, einer Dreieckform, einer Trapezform und einer Polygonalform besteht.

10. Verfahren zur Herstellung eines Spenderbehälters für Sondenabdeckungen von Ohrthermometern, wobei jede Sondenabdeckung einen hohlen Körper mit einem offenen Ende und einem gegenüberliegenden geschlossenen Ende aufweist, wobei ein Flansch sich radial von dem offenen Ende nach außen erstreckt, wobei das Verfahren die Schritte umfasst:
Bereitstellen eines flexiblen hohlen Hauptkörpers, der darauf ausgelegt ist, eine Vielzahl von gestapelten Sondenabdeckungen aufzunehmen, wobei eine erste Öffnung an einem Ende des hohlen Hauptkörpers ausgebildet ist, so dass das offene Ende der Sondenabdeckungen auf die erste Öffnung des hohlen Hauptkörpers ausgerichtet ist, wobei ein Boden an einem Ende des hohlen Hauptkörpers gegenüber von dem Ende, das die erste Öffnung aufweist, ausgebildet ist und wobei eine zweite Öffnung an einer Mitte des Bodens bereitgestellt ist; und
Bereitstellen eines Bodenbereichs, dessen eines Ende in dem Hohlraumkörper aufgenommen ist und dessen gegenüberliegendes Ende aus der zweiten Öffnung des hohlen Hauptkörpers hervorsteht, wobei der Bodenbereich eine dritte Öffnung aufweist, die auf die erste Öffnung ausgerichtet ist, zum Aufnehmen der hohlen Körper der Sondenabdeckungen, wobei sich ein Schulterbereich transversal von der dritten Öffnung zum Anliegen an dem Flansch der nächstliegenden Sondenabdeckung erstreckt und wobei der Schulterbereich auch an dem Boden des hohlen Hauptkörpers anliegt, um den Bodenbereich davon abzuhalten, durch die zweite Öffnung zu fallen, und wobei der Bodenbereich aus einem Material mit einer größeren Steifigkeit hergestellt ist als ein Material, aus dem der hohle Hauptkörper hergestellt ist, so dass ein Eingriff zwischen dem Bodenbereich und einer inneren Oberfläche des hohlen Hauptkörpers ausgebildet ist.

11. Spenderbehälter, der geeignet ist für Sondenabdeckungen (24) von Ohrthermometern, wobei jede Sondenabdeckung einen hohlen Körper (240) mit einem offenen Ende (241) und einem gegenüberliegenden geschlossenen Ende (242) aufweist, wobei ein Flansch (243) sich radial von dem offenen Ende (241) nach außen erstreckt, wobei der Spenderbehälter (30) einen hohlen Hauptkörper (41) und einen Bodenbereich (42) umfasst, wobei der Spenderbehälter (30) **dadurch gekennzeichnet ist, dass**
der hohle Hauptkörper (41) darauf ausgelegt ist, eine Vielzahl von gestapelten Sondenabdeckungen (24) aufzunehmen, wobei eine Öffnung (411) an einem Ende des hohlen Hauptkörpers (41) ausgebildet ist, so dass die offenen Enden (241) der Sondenabdeckung (24) auf die Öffnungen (411) des hohlen Hauptkörpers (41) ausgerichtet sein können, wobei ein Boden an einem anderen Ende des hohlen Hauptkörpers (41) ausgebildet ist gegenüber von dem Ende, das die Öffnung (411) des hohlen Hauptkörpers aufweist, wobei ein Paar von offenen Bereichen (43) jeweils an zwei lateralen Seiten des hohlen Hauptkörpers (41) ausgebildet sind; und dass
der Bodenbereich (42) in dem hohlen Hauptkörper (41) aufgenommen ist und in der Länge kleiner oder gleich groß wie der hohle Hauptkörper (41) ist, wobei der Bodenbereich (42) in dem hohlen Hauptkörper (41) beweglich ist, wobei der Bodenbereich (42) eine Öffnung (421) aufweist, die auf die Öffnung (411) des hohlen Hauptkörpers ausgerichtet ist, zum Aufnehmen der hohlen Körper der Sondenabdeckungen (24), wobei sich ein Schulterbereich (420) transversal von der Öffnung (421) des Bodenbereichs (42) zum Anliegen an dem Flansch (243) der nächstgelegenen Sondenabdeckung (24) erstreckt, wobei ein Paar von Griffbereichen (423) jeweils an zwei lateralen Seiten des Bodenbereichs bereitgestellt sind und aus den offenen Bereichen (43) des hohlen Hauptkörpers (41) entsprechend hervorstehen, wobei der hohle Hauptkörper (41) und der Bodenbereich aus einem flexiblen Material hergestellt sind und wobei ein Eingriff zwischen dem Bodenbereich (42) und der inneren Oberfläche des hohlen Hauptkörpers (41) ausgebildet ist.

12. Spenderbehälter nach Anspruch 11, wobei sich ein röhrenförmiger Bereich (322) von der dritten Öffnung (421) des Bodenbereichs erstreckt und wobei ein oberer Deckel (413) benachbart zu der ersten Öffnung (411) des hohlen Hauptkörpers (41) zum Schließen der ersten Öffnung bereitgestellt ist.

13. Spenderbehälter nach Anspruch 11, wobei jeder Griffbereich (423) des Bodenbereichs (42) eine Form aufweist, die aus der Gruppe gewählt ist, die aus einer Bogenform, einer Dreieckform, eine Rechteckform und einer Polygonalform besteht.

14. Spenderbehälter nach Anspruch 11, wobei der hohle Hauptkörper (41) eine Schnittform aufweist, die aus der Gruppe ausgewählt ist, die aus einer Rechteckform, einer Kreisform, einer Dreieckform, einer Trapezform und einer Polygonalform besteht.

15. Verfahren zur Herstellung eines Spenderbehalters für Sondenabdeckungen von Ohrthermometern, wobei jede Sondenabdeckung einen hohlen Körper mit einem offenen Ende und einem gegenüberliegenden geschlossenen Ende aufweist, wobei ein Flansch sich radial von dem offenen Ende nach außen erstreckt, wobei das Verfahren die Schritte umfasst:
Bereitstellen eines flexiblen hohlen Hauptkörpers, der darauf ausgelegt ist, eine Vielzahl an gestapelten Sondenabdeckungen aufzunehmen, wobei eine erste Öffnung an einem Ende des hohlen Hauptkörpers ausgebildet ist, so dass die offenen Enden der Sondenabdeckung auf die erste Öffnung des hohlen Hauptkörpers ausgerichtet sind, wobei ein Boden an einem Ende des hohlen Hauptkörpers gegenüber von dem Ende, das die erste Öffnung aufweist, ausgebildet ist und wobei ein Paar von offenen Bereichen jeweils an zwei lateralen Seiten des hohlen Hauptkörpers ausgebildet sind; und
Bereitstellen eines Bodenbereichs, der in dem hohlen Körper aufgenommen ist und der in der Länge kleiner oder gleich wie der hohle Hauptkörper ist, wobei es ihm möglich ist, sich innerhalb des hohlen Hauptkörpers zu bewegen, wobei der Bodenbereich eine dritte Öffnung aufweist, die auf die erste Öffnung ausgerichtet ist, zur Aufnahme der hohlen Körper der Sondenabdeckungen, wobei sich ein Schulterbereich transversal von der Öffnung zum Anliegen an dem Flansch der nächstgelegenen Sondenabdeckung erstreckt, wobei ein Paar von Griffbereichen an zwei lateralen Seiten des Bodenbereichs entsprechend bereitgestellt sind und wobei die Griffbereiche entsprechend aus den offenen Bereichen des hohlen Hauptkörpers hervorstehen, wobei der Bodenbereich aus einem Material hergestellt ist, das steifer ist als ein Material, aus dem der hohle Hauptkörper hergestellt ist, so dass ein Eingriff zwischen dem Bodenbereich und einer inneren Oberfläche des hohlen Hauptkörpers ausgebildet ist, so dass eine Reibung zwischen dem Bodenbereich und der inneren Oberfläche des hohlen Hauptkörpers größer ist als eine Reibung zwischen der inneren Oberfläche des hohlen Hauptkörpers und den Flanschen der Sondenabdeckungen.

## Revendications

1. Récipient distributeur approprié pour des couvre-sondes (24) de thermomètres auriculaires, chaque couvre-sonde (24) ayant un corps creux (240) avec une extrémité ouverte (241) et une extrémité fermée opposée (242), une bride (243) s'étendant dans le sens radial vers l'extérieur à partir de l'extrémité ouverte (241), le récipient distributeur (20) comprenant un corps principal creux (21) et une portion de fond (22), le récipient distributeur (20) étant **caractérisé en :**
**ce que** le corps principal creux (21) est configuré pour loger une pluralité de couvre-sondes empilés (24), une première ouverture (211) étant formée à une extrémité du corps principal creux (21) de telle manière que les extrémités ouvertes (241) des couvre-sondes (24) peuvent faire face à la première ouverture (211) du corps principal creux, une seconde ouverture (212) étant formée à une autre extrémité du corps principal creux (21) en face de l'extrémité ayant la première ouverture (211), au moins une paire de premières portions d'about (215) étant prévue à l'intérieur du corps principal creux (21) adjacent à la seconde ouverture (212) et
la portion de fond (22) ayant une extrémité logée dans le corps principal creux (21) et une extrémité opposée dépassant de la seconde ouverture (212) du corps principal creux (21), la portion de fond (22) ayant une troisième ouverture (221) faisant face à la première ouverture (211) pour loger les corps creux des couvre-sondes, la portion de fond (22) étant prévue à l'extérieur avec au moins une paire de secondes portions d'about (223) adjacentes à la troisième ouverture (221) pour buter sur une paire de premières portions d'about (215) de manière à empêcher la portion de fond (22) de sortir par la seconde ouverture (212), la portion de fond (22) étant de plus pourvue d'au moins une paire de troisièmes portions d'about (224) adjacentes à la troisième ouverture (221) pour buter sur la bride (243) du couvre-sonde le plus proche, l'extrémité de la portion de fond (22) en face de la troisième ouverture (221) étant formée comme un fond,
dans lequel le corps principal creux (21) et la portion de fond (22) sont faits en un matériau flexible et ont des formes de section sensiblement similaires et une interférence est formée entre une surface extérieure de la portion de fond (22) et une surface intérieure du corps principal creux (21).

2. Récipient distributeur selon la revendication 1 dans lequel la portion de fond (22) et le corps principal creux (21) ont des longueurs sensiblement égales.

3. Récipient distributeur selon la revendication 1 comprenant de plus une boîte de recouvrement (23) dans lequel la boîte de recouvrement (23) et la portion de fond (22) ont des longueurs sensiblement égales tandis que la boîte de recouvrement (23) et le corps principal creux (21) ont des formes périphériques sensiblement similaires.

4. Récipient distributeur selon la revendication 1 dans lequel la forme de section du corps principal creux (21) est sélectionnée dans le groupe comprenant une forme rectangulaire, une forme circulaire, une forme triangulaire, une forme trapézoïdale et une forme polygonale.

5. Procédé pour faire un récipient distributeur pour des couvre-sondes de thermomètres auriculaires, chaque couvre-sonde ayant un corps creux avec une extrémité ouverte et une extrémité fermée opposée, une bride s'étendant dans le sens radial vers l'extérieur à partir de l'extrémité ouverte, et le procédé comprenant les étapes de :
fournir un corps principal creux flexible avec une extrémité formée avec une première ouverture et une extrémité en face de la première extrémité formée avec une seconde ouverture, au moins une paire de premières portions d'about étant prévues à l'intérieur du corps principal creux adjacent à la seconde ouverture et le corps principal creux étant configuré pour loger une pluralité de couvre-sondes empilés si bien que l'extrémité ouverte des couvre-sondes fait face à la première ouverture du corps principal creux et
fournir une portion de fond flexible et souple dont l'une des extrémités est logée dans le corps principal creux et une extrémité opposée fait saillie de la seconde ouverture du corps principal creux, la portion de fond ayant une troisième ouverture faisant face à la première ouverture pour loger les corps creux des couvre-sondes, au moins une paire des secondes portions d'about étant prévue sur une surface extérieure de la portion de fond adjacente à la troisième ouverture pour buter contre la paire de premières portions d'about de manière à empêcher que la portion de fond s'échappe à travers la seconde ouverture, au moins une paire de troisièmes portions d'about étant prévue adjacente à la troisième ouverture pour buter contre la bride du couvre-sonde le plus proche, la portion de fond ayant un fond à une extrémité opposée à la troisième ouverture, la portion de fond et le corps principal creux ayant des formes de section sensiblement similaires et une interférence étant formée entre la surface extérieure de la portion de fond et une surface intérieure du corps principal creux.

6. Récipient distributeur approprié pour des couvre-sondes (24) de thermomètres auriculaires, chaque couvre-sonde (24) ayant un corps creux (240) avec une extrémité ouverte (241) et une extrémité fermée opposée (242), une bride (243) s'étendant dans le sens radial vers l'extérieur à partir de l'extrémité ouverte (241), le récipient distributeur (20) comprenant un corps principal creux (31) et une portion de fond (32), le récipient distributeur (30) étant **caractérisé en ;**
**ce que** le corps principal creux (31) est configuré pour loger une pluralité de couvre-sondes empilés, une première ouverture (311) étant formée à une extrémité du corps principal creux (31) de telle manière que les extrémités ouvertes (241) des couvre-sondes (24) peuvent faire face à la première ouverture (311) du corps principal creux, un fond étant formé à une autre extrémité du corps principal creux (31) en face de l'extrémité ayant la première ouverture (311), le fond étant formé de manière centrale avec une seconde ouverture (312) et
la portion de fond (32) ayant une extrémité logée dans le corps principal creux (31) et une extrémité opposée faisant saillie de la seconde ouverture (312) du corps principal creux (31), la portion de fond (32) ayant une troisième ouverture (321) faisant face à la première ouverture (311) pour loger les corps creux des couvre-sondes (24), une portion d'épaulement (320) s'étendant dans le sens transversal à partir de la troisième ouverture (321) pour buter sur la bride (243) du couvre-sonde le plus proche (24) et butant aussi sur le fond du corps principal creux (31) de manière à empêcher que la portion de fond (32) ne sorte par la seconde ouverture (312),
le corps principal creux (31) et la portion de fond (32) étant faits en un matériau flexible et une interférence étant formée entre la portion d'épaulement (320) de la portion de fond (32) et une surface intérieure du corps principal creux (31).

7. Récipient distributeur selon la revendication 6, la portion d'épaulement (320) de la portion de fond (32) et la première ouverture (311) du corps principal creux (31) ayant des formes sensiblement similaires.

8. Récipient distributeur selon la revendication 6, un piédestal (324) étant prévu dans le sens transversal à l'extrémité de la portion de fond (32) en face de la troisième ouverture, le piédestal (324) et le corps principal creux (31) ayant des formes périphériques sensiblement similaires, la portion de fond (32) et le corps principal creux (31) ayant des longueurs sensiblement égales.

9. Récipient distributeur selon la revendication 6, le corps principal creux (31) ayant une forme de section sélectionnée dans le groupe comprenant une forme rectangulaire, une forme circulaire, une forme triangulaire, une forme trapézoïdale et une forme polygonale.

10. Procédé pour faire un récipient distributeur pour des couvre-sondes de thermomètres auriculaires, chaque couvre-sonde ayant un corps creux avec une extrémité ouverte et une extrémité fermée opposée, une bride s'étendant dans le sens radial vers l'extérieur à partir de l'extrémité ouverte, et le procédé comprenant les étapes de :
fournir un corps principal creux flexible étant configuré pour loger une pluralité de couvre-sondes empilés, une première ouverture étant formée à une extrémité du corps principal creux de telle manière que les extrémités ouvertes des couvre-sondes peuvent faire face à la première ouverture du corps principal creux, un fond étant formé à une extrémité du corps principal creux en face de l'extrémité ayant la première ouverture et une seconde ouverture étant prévue à un centre du fond et de
fournir une portion de fond dont l'une des extrémités est logée dans le corps principal creux et une extrémité opposée dépassant de la seconde ouverture du corps principal creux, la portion de fond ayant une troisième ouverture faisant face à la première ouverture pour loger les corps creux des couvre-sondes, une portion d'épaulement s'étendant dans le sens transversal à partir de la troisième ouverture pour buter sur la bride du couvre-sonde le plus proche et butant aussi sur le fond du corps principal creux de manière à empêcher que la portion de fond ne s'échappe par la seconde ouverture et la portion de fond étant faite en un matériau plus rigide qu'un matériau dont est fait le corps principal creux si bien qu'une interférence est formée entre la portion de fond et une surface intérieure du corps principal creux.

11. Récipient distributeur approprié pour des couvre-sondes (24) de thermomètres auriculaires, chaque couvre-sonde (24) ayant un corps creux (240) avec une extrémité ouverte (241) et une extrémité fermée opposée (242), une bride (243) s'étendant dans le sens radial vers l'extérieur à partir de l'extrémité ouverte (241), le récipient distributeur (20) comprenant un corps principal creux (41) et une portion de fond (42), le récipient distributeur étant **caractérisé en :**
**ce que** le corps principal creux (41) est configuré pour loger une pluralité de couvre-sondes empilés (24), une ouverture (411) étant formée à une extrémité du corps principal creux (41) de telle manière que les extrémités ouvertes (241) des couvre-sondes (24) peuvent faire face à l'ouverture (411) du corps principal creux (41), un fond étant formé à une autre extrémité du corps principal creux (41) en face de l'extrémité ayant l'ouverture (411) du corps principal creux, une paire de portions ouvertes (43) étant formée respectivement sur les deux côtés latéraux du corps principal creux (41) et
la portion de fond (42) étant logée dans le corps principal creux (41) et étant de longueur plus petite ou égale au corps principal creux (41), la portion de fond (42) étant mobile à l'intérieur du corps principal creux (41), la portion de fond (42) ayant une ouverture (421) faisant face à l'ouverture (411) du corps principal creux pour loger les corps creux des couvre-sondes (24), une portion d'épaulement (420) s'étendant dans le sens transversal à partir de l'ouverture (421) de la portion de fond (42) pour buter sur la bride (243) du couvre-sonde le plus proche (24), une paire de portions de poignées (423) étant prévue respectivement sur les deux côtés latéraux de la portion de fond et faisant saillie respectivement des portions ouvertes (43) du corps principal creux (41), le corps principal creux (41) et la portion de fond étant faits en un matériau flexible et une interférence étant formée entre la portion de fond (42) et la surface intérieure du corps principal creux (41).

12. Récipient distributeur selon la revendication 11, une portion tubulaire (322) s'étendant à partir de la troisième ouverture (421) de la portion de fond et un couvercle supérieur (413) étant prévu adjacent à la première ouverture (411) du corps principal creux (41) pour fermer la première ouverture.

13. Récipient distributeur selon la revendication 11, chaque portion de poignée (423) mentionnée de la portion de fond (42) ayant une forme sélectionnée dans le groupe comprenant une forme rectangulaire, une forme circulaire, une forme triangulaire, une forme trapézoïdale et une forme polygonale.

14. Récipient distributeur selon la revendication 11, le corps principal creux (41) ayant une formé de section sélectionnée dans le groupe comprenant une forme rectangulaire, une forme circulaire, une forme triangulaire, une forme trapézoïdale et une forme polygonale.

15. Procédé pour faire un récipient distributeur pour des couvre-sondes de thermomètres auriculaires, chaque couvre-sonde ayant un corps creux avec une extrémité ouverte et une extrémité fermée opposée, une bride s'étendant dans le sens radial vers l'extérieur à partir de l'extrémité ouverte, et le procédé comprenant les étapes de :
fournir un corps principal creux flexible étant configuré pour loger une pluralité de couvre-sondes empilés, une première ouverture étant formée à une extrémité du corps principal creux de telle manière que les extrémités ouvertes des couvre-sondes peuvent faire face à la première ouverture du corps principal creux, un fond étant formé à une extrémité du corps principal creux en face de l'extrémité ayant la première ouverture et une paire de portions ouvertes étant formées sur deux côtés latéraux du corps principal creux et
fournir une portion de fond qui est logée dans le corps principal creux et dont la longueur est plus petite ou égale au corps principal creux qui permet de se déplacer à l'intérieur du corps principal creux, la portion de fond ayant une troisième ouverture faisant face à la première ouverture pour loger les corps creux des couvre-sondes, une portion d'épaulement s'étendant dans le sens transversal à partir de la troisième ouverture pour buter sur la bride du couvre-sonde le plus proche, une paire de portions de poignées étant prévue respectivement sur deux côtés latéraux de la portion de fond et les portions de poignée faisant saillie respectivement des portions ouvertes du corps principal creux, la portion de fond étant faite en un matériau plus rigide qu'un matériau dont est fait le corps principal creux si bien qu'une interférence est formée entre la portion de fond et une surface intérieure du corps principal creux et une friction entre la portion de fond et la surface intérieure du corps principal creux étant plus grande qu'une friction entre la surface intérieure du corps principal creux et les brides des couvre-sondes.
